# EUROPÄISCHE PATENTSCHRIFT

(11) **EP 3 357 352 B1**
(45) Veröffentlichungstag und Bekanntmachung des Hinweises auf die Patenterteilung: **24.03.2021**
(21) Anmeldenummer: 18155013.8
(22) Anmeldetag: 02.02.2018
(51) Int. Cl.: A23L 29/20, A23L 29/231, A23L 33/175, A23K 20/142, A23K 20/163, A23K 40/10, A61K 31/198, A61K 31/405, A61K 31/4172, A61K 31/715, A61K 31/717, A61K 31/721, A61K 31/722, A61K 31/732, A23P 10/40, A61P 17/02, A61P 25/24, A61P 37/02

(54) **ZUSAMMENSETZUNG MIT AMINOSÄUREN**
COMPOSITION WITH AMINO ACIDS
COMPOSITION COMPRENANT DES ACIDES AMINÉS

(30) Priorität: 02.02.2017 EP 17154477
(43) Veröffentlichungstag der Anmeldung: 08.08.2018
(73) Patentinhaber: artgerecht GmbH, 60313 Frankfurt (DE)
(72) Erfinder: Reheis, Daniel, 60318 Frankfurt (DE)
(74) Vertreter: K & H Bonapat Patentanwälte Koch · von Behren & Partner mbB

(56) Entgegenhaltungen:
- EP-A1- 0 030 448
- EP-A2- 1 330 957
- CH-A5- 569 426
- DE-A1- 4 128 260
- DE-A1-102007 034 102
- DE-A1-102011 000 997
- DE-U1-202012 011 540
- DE-U1-202013 104 575
- US-A1- 2013 090 297

## Beschreibung

Die Erfindung betrifft eine Zusammensetzung mit Aminosäuren, ein Verfahren zur Herstellung der Zusammensetzung und die Verwendung der Zusammensetzung als Nahrungsmittelzusatz und als Nahrungsergänzungsmittel.

Ein Vollnahrungsmittel für den Menschen, das neben einer Reihe anderer Bestandteile Aminosäuren enthält, ist in der DE 000001906763 C3 beschrieben.

Die DE 000060119919 T2 offenbart eine Zusammensetzung zur Behandlung einer beeinträchtigten Funktion des Magen-Darm-Trakts, die freie Aminosäuren einschließlich 9,0 bis 17,0 Gew.-% Glutaminsäure und 2,3 bis 10 Gew.-% Cystein und kein Glutamin enthält. Die beschriebene Zusammensetzung ist vorzugsweise zur Vollernährung geeignet und enthält Vitamine, Mineralstoffe, und energieliefernde Kohlenhydrate wie Maltodextrin, Maisstärke, Saccharose, Lactose, Fructose und Glucose.

In der US 2015/0306162 A1 wird eine pharmazeutische Zusammensetzung zur Behandlung von Darmdurchlässigkeit beschrieben, die Gummi arabicum, insbesondere in Kombination mit Fructo-Oligosacchariden, enthält. Die Zusammensetzung kann die Aminosäure L-Glutamin enthalten.

In der WO 01/43758 A1, WO 2004/026294 A1, WO 2004/110173 A1, WO 2007/069900 A1 und der WO 2014/007635 A1 werden Zusammensetzungen beschrieben, die neben vielen anderen Substanzen Aminosäuren und Polysaccharide enthalten.

In der CH 569 426 A5 wird eine Mischung von Aminosäuren und eines Kalorien liefernden Materials wie Fette oder Kohlenhydrate beschrieben, die einen Geschmackstoff und Pektin enthält. Das Pektin dient zur Verstärkung der durch geschmacksgebende Öle bewirkten Geschmacksüberdeckung, wodurch der Gehalt des Geschmacksstoffes in der Mischung verringert werden kann.

Die DE 10 2011 000997 A1 beschreibt Zusammensetzungen zu Ernährungszwecken für den Aufbau der Knochenmatrix, die Kollagenhydrolysat und Präbiotika enthalten. Danach kann der leimige Geschmack des Kollagenhydrolysats durch Präbiotika wie Inulin, Fruktane, Galakto-Oligosaccharide, Fructo-Oligosaccharide, resistente Maltodextrine und Polydextrose fast vollständig eliminiert werden. Das verwendete Kollagenhydrolysat weist ein mittleres Molekulargewicht im Bereich von ca. 600 bis ca. 18000 Da, insbesondere ca. 2500 bis 3500 Da, auf.

Ein Nahrungsergänzungsmittel mit Milcheiweiß oder Sojaeiweiß, Omega-3-Fettsäuren oder geschmacksneutrale Pflanzenöle, Aminosäuren, Vitamine, Mineralien, Spurenelemente, Ballaststoffe und Kohlenhydrate als Zutaten ist aus der EP 1 330 957 A2 bekannt. Bei diesem Grundrezept können die Aminosäuren bis auf 900g/kg angereichert werden, wobei Süßstoffe und Aromastoffe wegen des unangenehmen Geschmackes von Aminosäuren zugegeben werden. Wird das Nahrungsergänzungsmittel bei übergewichtigen Personen zum Fettabbau eingesetzt, so kann es Guarkernmehl als löslichen Ballaststoff enthalten, das die Magen-Darm-Passage unterstützen soll.

Die US 2013/090297 A1 beschreibt Zusammensetzungen für die Förderung der Muskelproteinsynthese und für den Einsatz bei Tumor-induziertem Gewichtsverlust, die freie Aminosäuren neben anderen Komponenten wie Protein, mehrfach ungesättigten Fettsäuren, Vitamine, Mineralien, Kohlenhydrate und Fette enthalten. Es werden lösliche Fasern wie Inulin, hydrolysierter Guargummi, Gummi arabicum und Bindemittel wie Tragacanth oder Methylcellulose ohne Nennung von Wirkungen erwähnt.

Aus der DE 10 2007 034 102 A1 sind Glycocyamin-haltige Formlinge mit einem Glycocyamingehalt von 55 bis 99,9 Gewichtsprozent, bis zu 40 Gewichtsprozent ernährunsphysiologisch wirksame Stoffe wie Kohlenhydrate, Fette, Aminosäuren, Proteine, Vitamine. Mineralstoffe und Spurenelemente bekannt. 0,05 bis 15 Gewichtsprozent eines Bindemittels, u.a. Cellulose-Derivate, Xanthan und Gummi arabicum werden genannt.

DE 20 2012 011 540 U1 offenbart eine Nahrungsmittelzusammensetzung mit Aminosäuren, z.B. Erbsenproteinhydrolysat, und Kakao. Polysaccharide sind nicht genannt.

In der DE 20 2013 104575 U1 wird eine mikroverkapselte Aminosäurenmischung mit Beschichtungsstoffen und Benetzungsstoffen, u.a. Gummi arabicum, beschrieben. Die Beschichtungsstoffe und Benetzungsstoffe verdecken den Geschmack der Aminosäuren.

DE 41 28 260 A1 und EP 0 030 448 A1 beschreiben Tabletten mit Aminosäuren, die Gummi arabicum im Überzug enthalten.

Problematisch bei der Verwendung von Zusammensetzungen, die größere Mengen von freien Aminosäuren enthalten, sind ein unerträglicher Geschmack und eine mangelnde Stabilität.

Aufgabe der Erfindung ist eine alternative Zusammensetzung mit Aminosäuren zu schaffen.

Die erfindungsgemäße Aufgabe wird durch eine Zusammensetzung mit den Merkmalen von Anspruch 1, ein Verfahren mit den Merkmalen von Anspruch 9 und die Verwendung mit den Merkmalen des Anspruchs 11 gelöst. In den abhängigen Ansprüchen sind vorteilhafte Ausgestaltungen der Erfindung angegeben.

Die Zusammensetzung enthält einen Aminosäureanteil (Aminosäurekomponente), einen Polysaccharidanteil (Polysaccharid-Komponente), optional L-Carnitin, Taurin oder Cholin und optional Zusatzstoffe oder Hilfsstoffe. Zusatzstoffe oder Hilfsstoffe sind in der Regel färbende Substanzen und gegebenenfalls Aromastoffe. Andere Substanzen oder Stoffe, insbesondere Proteine, Lipide, verdaubare Kohlenhydrate oder Nährstoffe, sind in der Regel nicht oder nur als Verunreinigung enthalten. Die Zusammensetzung dient zur Versorgung des Körpers mit einer oder mehreren Aminosäuren beziehungsweise Aminosäurearten, insbesondere in konzentrierter Form.

Der Aminosäureanteil ist mit einem Gewichtsanteil von mindestens 50 oder 60 Gewichtsprozent, bezogen auf die Trockenmasse der Zusammensetzung, in der Zusammensetzung enthalten. In der Regel ist der Aminosäureanteil mit einem Gewichtsanteil von 50 bis 90 Gewichtsprozent, vorzugsweise 60 bis 80 Gewichtsprozent, bezogen auf die Trockenmasse der Zusammensetzung, in der Zusammensetzung enthalten.

Die Zusammensetzung mit Aminosäure- und Polysaccharidanteil ist eine konzentrierte Aminosäure-Zusammensetzung, genauer eine Zusammensetzung mit einem Aminosäureanteil von mindestens 50 Gewichtsprozent, bezogen auf die Trockenmasse der Zusammensetzung, bzw. von mindestens 50% der Masse der Trockenmasse der Zusammensetzung. Die Zusammensetzung ist zur oralen Verabreichung bzw. zur Einnahme geeignet.

Vorzugsweise besteht die Zusammensetzung mindestens zu 95%, 96%, 97%, 98% oder 99% der Masse der Zusammensetzung (Trockenmasse) aus Aminosäureanteil, Polysaccharidanteil und optional einem, mehreren oder allen Stoffen aus der Gruppe der Stoffe L-Carnitin, Taurin und Cholin. Der Polysaccharidanteil, bestehend aus Gummi arabicum und Pektin, ist mit einem Gewichtsanteil von 1 bis 50 Gewichtsprozent, bezogen auf die Trockenmasse der Zusammensetzung, enthalten. Der Aminosäureanteil und der Polysaccharidanteil sind vermischt, als Mischung oder in einem Granulat enthalten.

Die Zusammensetzung ist vorzugsweise fest, insbesondere ein Pulver oder Granulat, kann aber auch flüssig, gelartig oder pastös sein. Die Zusammensetzung kann eine Flüssigkeit, insbesondere Wasser, enthalten.

Die Zusammensetzung enthält die Aminosäuren als freie Aminosäure oder deren Salz. Salze sind z.B. Hydrochloride. Der Zusammensetzung werden Proteine oder Peptide als Aminosäurequelle nicht zugesetzt.

Die Zusammensetzung enthält vorzugsweise mindestens zwei verschiedene Aminosäuren, besonders bevorzugt mindestens 5 verschiedene Aminosäuren, insbesondere mindestens 10, 11, 12, 13, 14, 15, 16, 17, 18, 19 oder 20 verschiedene Aminosäuren. Die in der Zusammensetzung enthaltenen Aminosäuren (freie Aminosäuren oder deren Salze; keine Aminosäuren in proteingebundener Form) bilden den Aminosäureanteil. Der Aminosäureanteil kann auch nur aus einer Aminosäureart bestehen.

Die Zusammensetzung enthält vorzugsweise die Aminosäuren: L-Glutamin, L-Leucin, L-Prolin, L-Lysin, L-Valin, L-Isoleucin, L-Tyrosin, L-Serin, L-Threonin, L-Alanin, L-Phenylalanin, L-Arginin, L-Histidin, Glycin, L-Cystein und/oder L-Cystin, L-Glutaminsäure, L-Tryptophan, L-Methionin, optional L-Asparaginsäure und optional L-Asparagin, wobei einzelne Aminosäuren als Salz vorliegen können. Beispielsweise besteht der Aminosäureanteil der Zusammensetzung im Wesentlichen oder vollständig aus den genannten Aminosäuren.

Vorzugsweise umfasst die Zusammensetzung eine, mehrere oder besonders bevorzugt alle der in Tabelle 1 aufgeführten Aminosäuren in den dort genannten Mengen, wobei L-Asparagin auch fehlen kann. Beispielsweise besteht der Aminosäureanteil der Zusammensetzung vollständig aus den in Tabelle 1 aufgeführten Aminosäuren (L-Asparagin optional). Der Aminosäureanteil bzw. das Aminosäureprofil (Art und Menge der Aminosäuren) der bevorzugten Zusammensetzung mit den in Tabelle 1 genannten Aminosäuren (L-Asparagin optional) wurde für ein optimales Wachstum und optimale Funktion aller Systeme und Organe im Kindesalter optimiert (optimiertes Aminosäureprofil). Eine Zusammensetzung mit einem solchen Aminosäureprofil eignet sich ideal als Nahrungsergänzungsmittel für Kleinkinder und Erwachsene.

Bezogen auf die Trockenmasse der Zusammensetzung enthält die Zusammensetzung vorzugsweise: 9 bis 17,5 Gewichtsprozent L-Glutamin, 5 bis 11 Gewichtsprozent L-Leucin, 3 bis 7 Gewichtsprozent L-Prolin, 2,6 bis 6,6 Gewichtsprozent L-Lysin HCl, 1,9 bis 6 Gewichtsprozent L-Valin, 1,7 bis 5,7 Gewichtsprozent L-Isoleucin, 1,9 bis 4,9 Gewichtsprozent L-Tyrosin, 1,7 bis 4,7 Gewichtsprozent L-Serin, 1,7 bis 4,7 Gewichtsprozent L-Threonin, 1,1 bis 4,1 Gewichtsprozent L-Alanin, 1 bis 4,1 Gewichtsprozent L-Phenylalanin, 0,9 bis 4 Gewichtsprozent L-Arginin, 1,2 bis 2 Gewichtsprozent L-Histidin, 1,2 bis 2,2 Gewichtsprozent Glycin, 1,2 bis 2,3 Gewichtsprozent L-Cystein, 0,7 bis 1,9 Gewichtsprozent L-Glutaminsäure, 0,7 bis 1,9 Gewichtsprozent L-Tryptophan, 0,2 bis 1,4 Gewichtsprozent L-Methionin, 0,7 bis 1,9 Gewichtsprozent L-Asparaginsäure, 0 bis 1,9 Gewichtsprozent L-Asparagin und optional 4 bis 12 Gewichtsprozent L-Carnitin, 0,8 bis 1,9 Gewichtsprozent Taurin und 1,5 bis 2,5 Gewichtsprozent Cholin (berechnet für Cholin-Bitartrat), wobei die Angaben der Gewichtsprozente sich auf die Trockenmasse der Zusammensetzung beziehen und unter der Maßgabe, dass die Summe der Gewichtsprozente, also der Aminosäureanteil, maximal einen Wert von 90 Gewichtsprozent, vorzugsweise maximal einen Wert von 80 Gewichtsprozent, insbesondere maximal einen Wert von 75 Gewichtsprozent, ergibt.

**Tabelle 1: Aminosäuregehalt (A allgemein, B bevorzugt, C besonders bevorzugt, D typischer Wert) des Aminosäureanteils der Zusammensetzung in Gewichtsprozent Gew.-% (bezogen auf den Aminosäureanteil; Gewichtsprozent ist die Anzahl Gramm, die in 100 g eines Gemisches enthalten ist)**

| Aminosäure | A) Gew.-% | | B) Gew.-% | | C) Gew.-% | | D) Gew.-% |
|---|---|---|---|---|---|---|---|
| | von | bis | von | bis | von | bis | |
| L-Glutamin | 13,7 | 25,6 | 17,7 | 21,6 | 18,7 | 20,7 | 19,7 |
| L-Leucin | 7,5 | 16,4 | 10,7 | 13,1 | 11,3 | 12,5 | 11,9 |
| L-Prolin | 4,5 | 10,4 | 6,7 | 8,2 | 7,1 | 7,8 | 7,5 |
| L-Lysin | 3,9 | 9,8 | 6,2 | 7,5 | 6,5 | 7,2 | 6,9 |
| L-Valin | 2,8 | 8,8 | 5,2 | 6,4 | 5,5 | 6,1 | 5,8 |
| L-Isoleucin | 2,5 | 8,5 | 5,0 | 6,1 | 5,2 | 5,8 | 5,5 |
| L-Tyrosin | 2,8 | 7,3 | 4,6 | 5,6 | 4,8 | 5,3 | 5,1 |
| L-Serin | 2,5 | 7,0 | 4,3 | 5,2 | 4,5 | 5,0 | 4,8 |
| L-Threonin | 2,5 | 7,0 | 4,3 | 5,2 | 4,5 | 5,0 | 4,8 |
| L-Alanin | 1,6 | 6,1 | 3,5 | 4,3 | 3,7 | 4,1 | 3,9 |
| L-Phenylalanin | 1,6 | 6,1 | 3,5 | 4,3 | 3,7 | 4,1 | 3,9 |
| L-Arginin | 1,5 | 6,0 | 3,4 | 4,1 | 3,5 | 3,9 | 3,7 |
| L-Histidin | 1,9 | 3,4 | 2,4 | 3,0 | 2,5 | 2,8 | 2,7 |
| Glycin | 1,8 | 3,3 | 2,3 | 2,8 | 2,4 | 2,7 | 2,5 |
| L-Cystein | 1,7 | 3,3 | 2,3 | 2,8 | 2,4 | 2,7 | 2,5 |
| L-Glutaminsäure | 1,1 | 2,8 | 1,7 | 2,1 | 1,8 | 2,0 | 1,9 |
| L-Tryptophan | 1,1 | 2,8 | 1,7 | 2,1 | 1,8 | 2,0 | 1,9 |
| L-Methionin | 0,3 | 2,0 | 1,1 | 1,3 | 1,1 | 1,3 | 1,2 |
| L-Asparaginsäure | 1,1 | 2,8 | 1,7 | 2,1 | 1,8 | 2,0 | 1,9 |
| L-Asparagin | 0 | 2,8 | 1,7 | 2,1 | 1,8 | 2,0 | 1,9 |

Tabelle 1 ist so zu verstehen, dass z.B. die Aminosäure L-Glutamin im Allgemeinen in einem Bereich A) von 13,7 bis 25,6 Gewichtsprozent, in einem bevorzugten Bereich B) von 17,7 bis 21,6 Gewichtsprozent, in einem besonders bevorzugten Bereich C) von 18,7 bis 20,7 Gewichtsprozent und mit einem typischen Wert D (Beispielwert) von 19,7 Gewichtsprozent, bezogen auf den Aminosäureanteil, in dem Aminosäureanteil der Zusammensetzung enthalten ist. Entsprechendes gilt für die anderen aufgeführten Aminosäuren. Die Angaben in Tabelle 1 sind Beispiele für den Gehalt einer einzelnen, mehrerer oder aller Aminosäuren der Zusammensetzung. Ein niedriger Anteil an Arginin (z.B. etwa 1 bis 6 Gew.-% der Gesamtaminosäuren) stimuliert die endogene Produktion von Glutamin. Leucin stimuliert die Muskelproteinsynthese. Tryptophan fördert eine Erhöhung der Proteinsynthese. Lysin ist ein wichtiger Baustein wichtiger Körpersubstanzen wie Proteine und Enzyme.

Ein Mengenanteil von Leucin von bis zu 11 Gewichtsprozent und ein Mengenanteil von Methionin von bis zu 1,4 Gewichtsprozent in der Zusammensetzung erwiesen sich hinsichtlich der Verwendung als Nahrungsergänzungsmittel als günstig. Besonders günstig erwies sich ein Gewichtsverhältnis der Aminosäuren Leucin und Glycin mit einem Wert im Bereich von 4,5:1 bis 5:1 (Gewicht Leucin : Gewicht Glycin). Ferner erwies sich ein Gewichtsverhältnis der Aminosäuren Methionin und Glycin mit einem Wert im Bereich von 0,45:1 bis 0,5:1 (Gewicht Methionin : Gewicht Glycin) als günstig. Ferner erwies sich ein Gewichtsverhältnis der Aminosäuren Leucin, Methionin und Glycin zueinander, Leucin/Methionin/Glycin von etwa 10:1:2 in der Zusammensetzung als physiologisch günstig.

Die Zusammensetzung enthält optional L-Carnitin, Cholin (z.B. als Cholin-bitartrat oder Cholinchlorid), Taurin (2-Aminoethansulfonsäure) oder deren chemische Äquivalente (Salze oder Derivate). Bevorzugt sind L-Carnitin und Taurin oder L-Carnitin, Cholin und Taurin (oder deren chemische Äquivalente) in der Zusammensetzung enthalten. Diese Stoffe ergänzen vorteilhaft die Aminosäurekomponente der Zusammensetzung, gehören aber nicht zum Aminosäureanteil.

Von dem Begriff "Polysaccharid" sind Substanzen, die Polysacchariden ähnlich sind, Polysaccharid-artige Substanzen, Polysaccharid-Derivate oder von einem Polysaccharid durch chemische Reaktion abgeleitete oder umgewandelte Polymere mit umfasst. Die Begriffe Polysaccharid-Komponente und Polysaccharidanteil stehen für die Gesamtheit der in der Zusammensetzung enthaltenen Polysaccharide, einschließlich der Polysaccharid-Derivate, Polysaccharid-artigen Verbindungen und Polysaccharid-ähnlichen Verbindungen. Von dem Begriff "Polysaccharidanteil" sind auch Substanzen umfasst, die Polysaccharide als Hauptbestandteil und Beimengungen von anderen Stoffen enthalten. Solche Substanzen sind in der Regel Bioprodukte, natürliche Produkte oder aus natürlichen Quellen hergestellte Produkte, z.B. Pflanzengummi oder Pflanzenextrakte. Solche Substanzen sind auch Rohprodukte oder Polysaccharid-Präparationen pflanzlicher Herkunft. Polysaccharide sind in der Regel Kohlenhydrate, in denen eine große Anzahl (mindestens zehn, vorzugsweise mindestens dreißig, besonders bevorzugt mindestens fünfzig) von Polysaccharid-bildenden Monomeren wie Monosaccharide, Monosaccharid-ähnliche Verbindungen oder Monosaccharid-Derivate verbunden sind.

Die in der Zusammensetzung enthaltenen Polysaccharide sind löslich bzw. sind in einem wässrigen Medium oder in Wasser löslich, teilweise löslich, nehmen Wasser auf, quellen in Wasser oder bilden Hydrokolloide oder Gele. Solche Polysaccharide sollen hier von dem Begriff "lösliche Polysaccharide" mit umfasst sein. Substanzen, die lösliche Polysaccharide enthalten, sind beispielsweise Produkte, die aus Pflanzen gewonnen werden und hauptsächlich aus einem Polysaccharid oder Polysacchariden bestehen.

Pektine sind Bestandteil des Polysaccharidanteils. Beispielsweise geben sie der Zusammensetzung eine günstige Konsistenz, wobei durch die Wahl des Pektins aus der weiten Gruppe der Pektine, die sich in Molekülgröße und Veresterungsgrad in einem weiten Bereich unterscheiden, die Konsistenz der Zusammensetzung variiert werden kann. Ernährungsphysiologisch betrachtet sind Pektine für den Menschen Ballaststoffe. Viele Mikroorganismen dagegen sind in der Lage, Pektine zu fermentieren. Pektine sind als Bestandteil einer nährstoffreduzierten Zusammensetzung sehr vorteilhaft.

Gummi arabicum ist ein natürliches Gemisch von Polysacchariden und wird aus dem Pflanzensaft von Verek-Akazien (Senegalia senegal) und Seyal-Akazien (Vachellia seyal) gewonnen. Gummi arabicum wird auch als Akaziengummi (Acacia Gum) bezeichnet. Vorteilhaft wird hier Gummi arabicum oder Akaziengummi in Faserform eingesetzt (Akazienfaser). Akazienfaser ist unter dem Handelsnamen Fibregum© von der Firma Nexira in Rouen (Frankreich) erhältlich. Gummi arabicum, insbesondere Akazienfaser, erwies sich als sehr vorteilhaft zur Verwendung als Polysaccharid-Komponente oder als Bestandteil des Polysaccharidanteils. Vorteilhaft sind Mischungen von Gummi arabicum, insbesondere Akazienfaser, und Pektinen, insbesondere Obstpektine mit faseriger Struktur. Eine faserige Struktur von solchen Produkten ist besonders vorteilhaft für die Herstellung von porösen oder Hohlraum-reichen Granulaten der Zusammensetzung. Solche Granulate führen zu einem angenehmen Mundgefühl bei der Einnahme.

Mischungen von Polysacchariden für den Polysaccharidanteil sind Mischungen von Pektin und Gummi arabicum. Je nach Mischung können sich günstige Eigenschaften ergänzen oder verstärken. Außerdem kann durch Einsatz von Polysaccharid-Mischungen die Konsistenz und die Löseeigenschaft der Zusammensetzung leicht variiert werden, wodurch die Zusammensetzung für die jeweilige Verwendung angepasst werden kann.

Der Polysaccharidanteil der Zusammensetzung beträgt vorzugsweise 5 bis 50 Gewichtsprozent der Zusammensetzung, insbesondere 10 bis 45 Gewichtsprozent der Zusammensetzung, und der Anteil von Hilfs- und Zusatzstoffen beträgt in der Regel 0 bis 5 Gewichtsprozent der Zusammensetzung, vorzugsweise 0 bis 3 Gewichtsprozent der Zusammensetzung, insbesondere 0 bis 1 Gewichtsprozent der Zusammensetzung, wobei die Angaben der Gewichtsprozente sich auf die Trockenmasse der Zusammensetzung beziehen und die Summe der Gewichtsprozente von Aminosäureanteil, Polysaccharidanteil, Hilfsstoffe, Zusatzstoffe, L-Carnitin, Taurin und Cholin bzw. ein Cholin-Salz, also aller Bestandteile der Zusammensetzung, maximal einen Wert von 100 Gewichtsprozent ergibt..

Die feste Zusammensetzung ist in der Regel wasserlöslich, wobei die Löslichkeit der Zusammensetzung in der Regel vom Polysaccharidanteil bestimmt wird.

Vorteilhaft ist eine Zusammensetzung, erhältlich durch Mischen der festen Zusammensetzung mit einer Flüssigkeit, vorzugsweise eine wässrige Flüssigkeit wie Wasser oder eine wässrige Lösung. Die erhaltene Zusammensetzung kann z.B. eine Lösung, eine Suspension oder feindisperse Mischung sein.

Die Zusammensetzung ist kein Vollnahrungsmittel. Sie enthält nur den Aminosäureanteil, den Polysaccharidanteil und optional: Hilfsstoffe, Zusatzstoffe; L-Carnitin, Taurin und/oder Cholin bzw. ein Cholin-Salz wie Cholin-bitartrat oder Cholin-Chlorid.

Optionale Hilfs- oder Zusatzstoffe sind z.B. natürliches Aroma oder natürliche Farbstoffe wie Carotin oder Saft der Rote Beete (getrocknet oder flüssig).

Die Zusammensetzung enthält in der Regel keine oder im Wesentlichen keine Proteine oder Peptide. Sie können als Verunreinigung vorhanden sein. Herstellungsbedingt können geringe Mengen Protein in der Zusammensetzung enthalten sein. Proteine oder Peptide werden der Zusammensetzung nicht als Aminosäurequelle zugesetzt. Wenn überhaupt vorhanden, haben Proteine und Peptide zusammen einen Anteil von weniger als 3 Gewichtsprozent, vorzugsweise weniger als 2 Gewichtsprozent, insbesondere weniger als 1 Gewichtsprozent, bezogen auf die Trockenmasse der Zusammensetzung.

Die Zusammensetzung enthält in der Regel keine oder im Wesentlichen keine Vitamine.

Die Zusammensetzung enthält in der Regel keine oder im Wesentlichen keine Mineralstoffe oder Mineralien. Geringe Mengen von anorganischen Salzen sind möglich, in der Regel weniger als 1 Gewichtsprozent, vorzugsweise weniger als 0,5 Gewichtsprozent und besonders bevorzugt weniger als 0,1 Gewichtsprozent.

Die Zusammensetzung enthält in der Regel keine oder im Wesentlichen keine Lipide.

Die Zusammensetzung enthält vorzugsweise außer den färbenden Substanzen und Aromastoffen als Zusatz- oder Hilfsstoffe keine chemischen Zusätze und Süßungsmittel. Sollte die Zusammensetzung Beimengungen von Proteinen, Peptiden, Vitaminen, Lipiden, Mineralstoffen und Mineralien enthalten, so sind diese Beimengungen in der Regel durch die Herstellung der Bestandteile oder durch die Art der verwendeten Ausgangsstoffe bedingt. Insbesondere werden bei Verwendung von Naturprodukten oder Naturstoffen zur Herstellung der Zusammensetzung solche Beimengungen eingebracht. Sie werden als Verunreinigung betrachtet.

Die Zusammensetzung, insbesondere die Zusammensetzung mit bevorzugtem Aminosäureprofil (siehe Tabelle 1), wird vorteilhaft verwendet als Nahrungsmittelzusatz, als Nahrungsergänzungsmittel, als Diät-Zusatzmittel, als diätetische Zubereitung, als Sportlernahrungsergänzung, als Sportlernahrungsergänzungsmittel, als Babynahrungsergänzungsmittel, als Zusatz zu Babynahrung und als Mittel zur körperlichen Leistungssteigerung.

Die Zusammensetzung mit optimiertem Aminosäureprofil (siehe Tabelle 1) wird zur Leistungssteigerung, insbesondere im Leistungssport, eingesetzt. Leistungssportler verbrauchen durchschnittlich 1,7 Gramm Eiweiß pro Tag, wobei die Art von Aminosäuren ihre Leistung und Verletzungsgefahr signifikant beeinflusst. Die Zusammensetzung ist eine dafür angepasste Aminosäure-Quelle, wodurch die Muskulatur, das Gehirn, das Immunsystem und die inneren Organe mit den richtigen Aminosäuren im richtigen Verhältnis versorgt werden.

Die Zusammensetzung kann einem Nahrungsmittel oder einem Nahrungsergänzungsmittel zugesetzt werden. Vorteilhaft ist die Zusammensetzung, insbesondere die bevorzugte Zusammensetzung mit optimiertem Aminosäureprofil (siehe Tabelle 1), in Babynahrung enthalten oder dient als Zusatz für Babynahrung. Eine orale Verabreichung oder Einnahme konzentrierter Aminosäure-Zusammensetzungen wurde bisher durch einen üblen Geschmack ausgeschlossen. Mittels der erfindungsgemäßen Zusammensetzungen können nun Zusammensetzungen mit hoher Aminosäure-Konzentration eingenommen und im Mund verteilt werden.

Verwendet wird ein Polysaccharidanteil von 1 bis 50 Gewichtsprozent (bezogen auf die Trockenmasse der Zusammensetzung) aus Pektin und Gummi arabicum, Akaziengummi oder Akaziengummifaser, zur Verbesserung des Geschmackes einer Aminosäure-haltigen Zusammensetzung oder zur Verbesserung der Resorption von Aminosäuren einer Aminosäure-haltigen Zusammensetzung im Mund und Magen-Darm-Trakt, wobei die Zusammensetzung Aminosäuren enthält mit einem Gewichtsanteil (bezogen auf die Trockenmasse der Zusammensetzung) von mindestens 50 Gewichtsprozent Aminosäuren, wobei die Angaben zu den Gewichtsprozenten auf die Trockenmasse der gesamten Zusammensetzung bezogen sind.

### Herstellung der Zusammensetzung

Die Aminosäuren des Aminosäureanteils können durch Hydrolyse von Proteinen, vorzugsweise Pflanzenproteine, gewonnen werden.

Vorzugsweise werden Aminosäuren verwendet, die durch Fermentation gewonnen werden. Ein Verfahren zur Herstellung von L-Aminosäuren durch Fermentation wird beispielsweise in der DE 69833469 T2 beschrieben.

Bei dem Verfahren gemäß der Erfindung wird Granulat der Zusammensetzung mittels Wirbelschichtgranulation hergestellt. Dazu wird z.B. eine pulverige Ausgangsmischung angefeuchtet. Es kann z.B. auch zur Granulation einer pulverigen Mischung von Aminosäuren und Zusatzstoffen der Polysaccharidanteil mit Wasser als flüssiges oder wasserhaltiges Bindemittel zugegeben werden. Das Granulat bietet einen besonderen Vorteil gegenüber einer Zusammensetzung in Pulverform. Die Mischung "zergeht" auf der Zunge, wo hingegen ein Pulver sich "staubig" im Mund anfühlt und zur Klumpen-bildung neigt. Dadurch kann die als Granulat vorliegende Zusammensetzung direkt pur mit einem angenehmen Mundgefühl eingenommen werden.

Eine besonders porenhaltige oder hohlraumreiche feste Zusammensetzung lässt sich herstellen durch Verwendung löslicher Polysaccharide oder Substanzen mit löslichen Polysacchariden als Bestandteil, die eine faserige Struktur haben, z.B. Akazienfaser und Obstpektin. Diese Fasern werden mit Wasser so angefeuchtet, dass die Faserstruktur erhalten bleibt und die Oberfläche der Faser klebrig wird. Dies lässt sich z.B. durch Einwirkung von Wasserdampf erreichen. Das Anfeuchten kann bei einer fertigen pulverigen Mischung, die bereits alle Bestandteile der Zusammensetzung enthält, erfolgen. Die angefeuchtete Mischung wird dann vorzugsweise mit Hilfe der Wirbelschichtgranulation in ein Granulat überführt.

Es kann auch ein faseriges lösliches Polysaccharid oder eine Substanz, die ein faseriges lösliches Polysaccharid enthält, in Kombination mit Wasser als Bindemittel für die Granulation einer pulverigen Mischung der Zusammensetzung verwendet werden, die ein Polysaccharid bzw. einen Polysaccharidanteil bereits enthält. Faseriges lösliches Polysaccharid und der Polysaccharidanteil können gleichartig oder verschieden sein. Die Verwendung von faserigem löslichen Polysaccharid erlaubt die Herstellung von Granulat der Zusammensetzung mit besonderer Porenstruktur.

### Beispiel 1

Eine bevorzugte Zusammensetzung enthält:
a) Aminosäureanteil: 13,2 Gew.-% L-Glutamin, 8 Gew.-% L-Leucin, 5 Gew.-% L-Prolin, 4,6 Gew.-% L-Lysin HCl (Hydrochlorid), 3 Gew.-% L-Valin, 3,7 Gew.-% L-Isoleucin, 3,4 Gew.-% L-Tyrosin, 3,2 Gew.-% L-Serin, 3,2 Gew.-% L-Threonin, 2,6 Gew.-% L-Alanin, 2,6 Gew.-% L-Phenylalanin, 2,5 Gew.-% L-Arginin, 1,8 Gew.-% L-Histidin, 1,7 Gew.-% Glycin, 1,7 Gew.-% L-Cystein, 1,3 Gew.-% L-Glutaminsäure, 1,3 Gew.-% L-Tryptophan, 0,8 Gew.-% L-Methionin, 1,3 Gew.-% L-Asparaginsäure;
b) 8 Gew.-% L-Carnitin, 2 Gew.-% Cholin-Bitartrat, 1,4 Gew.-% Taurin;
c) Polysaccharidanteil: 11,5 Gew.-% Akazienfaser und 11,5 Gew.-% faseriges Obstpektin;
d) Rest Hilfsstoffe und Zusatzstoffe: natürliches Aroma, getrockneter Rote Beete-Saft (natürlicher Farbstoff);
wobei alle Bestandteile der Zusammensetzung zusammen 100 Gew.-% (Gewichtsprozent) ergeben.

Die Zusammensetzung liegt als trockenes, poröses Granulat vor und wird z.B. als Nahrungsergänzungsmittel oder diätetisches Mittel verwendet.

### Beispiel 2

Eine bevorzugte Zusammensetzung enthält:
a) Aminosäureanteil: 9 Gew.-% L-Glutamin, 8 Gew.-% L-Leucin, 7 Gew.-% L-Prolin, 3 Gew.-% L-Lysin HCl (Hydrochlorid), 2 Gew.-% L-Valin, 3 Gew.-% L-Isoleucin, 2 Gew.-% L-Tyrosin, 5 Gew.-% L-Serin, 3 Gew.-% L-Threonin, 2 Gew.-% L-Alanin, 2 Gew.-% L-Phenylalanin, 2 Gew.-% L-Arginin, 1,5 Gew.-% L-Histidin, 3 Gew.-% Glycin, 3 Gew.-% L-Cystein, 0,5 Gew.-% L-Glutaminsäure, 1,1 Gew.-% L-Tryptophan, 1,2 Gew.-% L-Methionin, 1 Gew.-% L-Asparaginsäure;
b) 5 Gew.-% L-Carnitin;
c) Polysaccharidanteil 30,0 Gew.-% Pektin und 5,0 Gew.-% Gummi arabicum;
d) Rest Hilfsstoffe und Zusatzstoffe, z.B. färbende Substanzen;
wobei alle Bestandteile der Zusammensetzung zusammen 100 Gew.-% (Gewichtsprozent) ergeben.

Die Zusammensetzung kann durch Zumischen von Wasser unter Erwärmung in ein Gel überführt werden.

## Patentansprüche

1. Zusammensetzung, die Aminosäuren und Polysaccharide enthält und zur oralen Verabreichung geeignet ist, **dadurch gekennzeichnet, dass** die Trockenmasse der Zusammensetzung besteht aus
a) einem Aminosäureanteil mit mindestens einer Aminosäure oder mit einer oder mehreren Aminosäuren ausgewählt aus der Gruppe L-Glutamin, L-Leucin, L-Prolin, L-Lysin, L-Valin, L-Isoleucin, L-Tyrosin, L-Serin, L-Threonin, L-Alanin, L-Phenylalanin, L-Arginin, L-Histidin, Glycin, L-Cystein, L-Cystin, L-Glutaminsäure, L-Tryptophan, L-Methionin, L-Asparagin und L-Asparaginsäure, wobei der Aminosäureanteil aus Aminosäuren besteht und die Aminosäuren als freie Aminosäure oder als deren Salz vorliegen,
b) einem Polysaccharidanteil, der aus Gummi arabicum und Pektin besteht,
c) optional einem, mehreren oder allen Stoffen aus der Gruppe der Stoffe L-Carnitin, Taurin oder Cholin, als solche, als Salz, als eine Vorstufe oder als ein Derivat, und
d) optional einem oder mehreren Hilfsstoffen oder Zusatzstoffen,
wobei der Aminosäureanteil mit einem Gewichtsanteil von mindestens 50 Gewichtsprozent und der Polysaccharidanteil mit einem Gewichtsanteil von 1 bis 50 Gewichtsprozent, jeweils bezogen auf die Trockenmasse der Zusammensetzung, enthalten sind und wobei der Aminosäureanteil und der Polysaccharidanteil vermischt sind, der Aminosäureanteil und der Polysaccharidanteil als Mischung oder in einem Granulat enthalten sind.

2. Zusammensetzung nach Anspruch 1, **dadurch gekennzeichnet, dass** die Zusammensetzung besteht zu mindestens 95 Gewichtsprozent aus dem Aminosäureanteil, dem Polysaccharidanteil und
optional einem oder mehreren Stoffen aus der Gruppe der Stoffe L-Carnitin, Taurin oder Cholin, als solche, als Salz, als eine Vorstufe oder als ein Derivat,
wobei der Aminosäureanteil mit einem Gewichtsanteil von 50 bis 90 Gewichtsprozent in der Zusammensetzung enthalten ist und die Angaben der Gewichtsprozente sich auf die Trockenmasse der Zusammensetzung beziehen.

3. Zusammensetzung nach einem der vorhergehenden Ansprüche, **dadurch gekennzeichnet, dass** Hilfs- und Zusatzstoffe mit einem Gewichtsanteil von 0 bis 5 Gewichtsprozent in der Zusammensetzung enthalten sind, wobei die Angaben der Gewichtsprozente sich auf die Trockenmasse der Zusammensetzung beziehen.

4. Zusammensetzung nach einem der vorhergehenden Ansprüche, **dadurch gekennzeichnet, dass** Aromastoffe oder färbende Substanzen als Hilfs- und Zusatzstoffe in der Zusammensetzung enthalten sind.

5. Zusammensetzung nach einem der vorhergehenden Ansprüche, **dadurch gekennzeichnet, dass** der Aminosäureanteil aus den Aminosäuren L-Glutamin, L-Leucin, L-Prolin, L-Lysin, L-Valin, L-Isoleucin, L-Tyrosin, L-Serin, L-Threonin, L-Alanin, L-Phenylalanin, L-Arginin, L-Histidin, Glycin, L-Cystein oder L-Cystin oder L-Cystein und L-Cystin, L-Glutaminsäure, L-Tryptophan, L-Methionin, optional L-Asparaginsäure und optional L-Asparagin besteht.

6. Zusammensetzung nach einem der vorhergehenden Ansprüche, **dadurch gekennzeichnet, dass** die Zusammensetzung 9 bis 17,5 Gewichtsprozent L-Glutamin, 5 bis 11 Gewichtsprozent L-Leucin, 3 bis 7 Gewichtsprozent L-Prolin, 2,6 bis 6,6 Gewichtsprozent L-Lysin HCl, 1,9 bis 6 Gewichtsprozent L-Valin, 1,7 bis 5,7 Gewichtsprozent L-Isoleucin, 1,9 bis 4,9 Gewichtsprozent L-Tyrosin, 1,7 bis 4,7 Gewichtsprozent L-Serin, 1,7 bis 4,7 Gewichtsprozent L-Threonin, 1,1 bis 4,1 Gewichtsprozent L-Alanin, 1 bis 4,1 Gewichtsprozent L-Phenylalanin, 0,9 bis 4 Gewichtsprozent L-Arginin, 1,2 bis 2 Gewichtsprozent L-Histidin, 1,2 bis 2,2 Gewichtsprozent Glycin, 1,2 bis 2,3 Gewichtsprozent L-Cystein, 0,7 bis 1,9 Gewichtsprozent L-Glutaminsäure, 0,7 bis 1,9 Gewichtsprozent L-Tryptophan, 0,2 bis 1,4 Gewichtsprozent L-Methionin und optional 4 bis 12 Gewichtsprozent L-Carnitin, 0,8 bis 1,9 Gewichtsprozent Taurin und 1,5 bis 2,5 Gewichtsprozent Cholin enthält, wobei die Angaben der Gewichtsprozente sich auf die Trockenmasse der Zusammensetzung beziehen und die Summe der Gewichtsprozente maximal einen Wert von 90 Gewichtsprozent ergibt.

7. Zusammensetzung nach einem der vorhergehenden Ansprüche, **dadurch gekennzeichnet, dass** die Zusammensetzung fest, als Pulver, als Granulat, als Lösung, als Suspension, als flüssige Dispersion, gelartig oder pastös vorliegt oder eine Flüssigkeit oder Wasser enthält.

8. Zusammensetzung nach einem der vorhergehenden Ansprüche, **dadurch gekennzeichnet, dass** die Zusammensetzung Proteine, Peptide, verdaubare Kohlenhydrate, Süßungsmittel, Lipide, Vitamine, Mineralstoffe oder Mineralien nicht oder nur als Verunreinigung enthält.

9. Verfahren zur Herstellung einer Zusammensetzung nach Anspruch 1 bis 8 **dadurch gekennzeichnet, dass** die Zusammensetzung mit Hilfe einer Wirbelschichtgranulation in eine poröse Form, einen porösen Feststoff oder ein poröses Granulat überführt wird.

10. Verfahren zur Herstellung einer Zusammensetzung nach Anspruch 9, **dadurch gekennzeichnet, dass** Mischungen von Gummi arabicum und Pektinen eingesetzt werden und solche Produkte eine faserige Struktur haben.

11. Verwendung einer Zusammensetzung nach Anspruch 1 bis 8, **dadurch gekennzeichnet, dass** die Zusammensetzung als Nahrungsmittelzusatz, als Nahrungsergänzungsmittel, als Diät-Zusatzmittel, als diätetische Zubereitung, als Sportlernahrungsergänzung, als Sportlernahrungsergänzungsmittel, als Babynahrungsergänzungsmittel, als Zusatz zu Babynahrung, zur körperlichen Leistungssteigerung oder zur vorbeugenden Ernährung verwendet wird.

## Claims

1. Composition that contains amino acids and polysaccharides and is suited for oral administration, **characterized in that** the dry mass of the composition consists of
a) an amino acid component with at least one amino acid or with one or more amino acids selected from the group L-glutamine, L-leucine, L-proline, L-lysine, L-valine, L-isoleucine, L-tyrosine, L-serine, L-threonine, L-alanine, L-phenylalanine, L-arginine, L-histidine, glycine, L-cysteine, L-cystine, L-glutamic acid, L-tryptophan, L-methionine, L-asparagine and L-aspartic acid, where the amino acid component consists of amino acids and the amino acids are present as free amino acids or as their salts,
b) a polysaccharide component consisting of gum arabic and pectin,
c) optionally one, several or all substances from the group of substances L-carnitine, taurine or choline, as such, as a salt, as a precursor or as a derivative, and
d) optionally one or more auxiliaries or additives,
wherein the content of the amino acid component is at least 50 percent by weight and the content of the polysaccharide component has 1 to 50 percent by weight, based on the dry weight of the composition, and wherein the amino acid component and the polysaccharide component are mixed, the amino acid component and the polysaccharide component are contained as a mixture or in a granulate.

2. Composition according to claim 1, **characterized in that** the composition consists of at least 95 percent by weight of the amino acid component, the polysaccharide component and optionally one or more substances from the group of substances L-carnitine, taurine or choline, as such, as a salt, as a precursor or as a derivative, wherein the content of the amino acid component in the composition is 50 to 90 percent by weight and the percentages by weight are based on the dry weight of the composition.

3. Composition according to one of the preceding claims, **characterized in that** auxiliaries and additives are present in the composition with a content of 0 to 5 percent by weight, where the percentages by weight are based the dry weight of the composition.

4. Composition according to one of the preceding claims, **characterized in that** flavorings or coloring substances are contained in the composition as auxiliaries and additives.

5. Composition according to one of the preceding claims, **characterized in that** the amino acid component consists of the amino acids L-glutamine, L-leucine, L-proline, L-lysine, L-valine, L-isoleucine, L-tyrosine, L-serine, L-threonine, L-alanine, L-phenylalanine, L-arginine, L-histidine, glycine, L-cysteine or L-cystine or L-cysteine and L-cystine, L-glutamic acid, L-tryptophan, L-methionine, optionally L-aspartic acid and optionally L-asparagine.

6. Composition according to one of the preceding claims, **characterized in that** the composition contains 9 to 17.5 percent by weight L-glutamine, 5 to 11 percent by weight L-leucine, 3 to 7 percent by weight L-proline, 2.6 to 6.6 percent by weight L-Lysine HCI, 1.9 to 6 percent by weight L-valine, 1.7 to 5.7 weight percent L-isoleucine, 1.9 to 4.9 weight percent L-tyrosine, 1.7 to 4.7 percent by weight L-serine, 1.7 to 4.7 percent by weight L-threonine, 1.1 to 4.1 percent by weight L-alanine, 1 to 4.1 percent by weight L-phenylalanine, 0.9 to 4 percent by weight L-arginine, 1.2 to 2 percent by weight L-histidine, 1.2 to 2.2 percent by weight glycine, 1.2 to 2.3 percent by weight L-cysteine, 0.7 to 1.9 percent by weight L-glutamic acid, 0.7 to 1.9 percent by weight L-tryptophan, 0.2 to 1.4 percent by weight of L-methionine and optionally 4 to 12 percent by weight of L-carnitine, 0.8 to 1.9 percent by weight of taurine and 1.5 to 2.5 percent by weight of choline, where the percentages by weight are based on the dry weight of the composition and the sum of the percentages by weight gives a value of up to 90 percent by weight.

7. Composition according to one of the preceding claims, **characterized in that** the composition is solid, is present as a powder, as granules, as a solution, as a suspension, as a liquid dispersion, is gel-like or pasty or contains a liquid or water.

8. Composition according to one of the preceding claims, **characterized in that** the composition contains proteins, peptides, digestible carbohydrates, sweeteners, lipids, vitamins, mineral substances or minerals not at all or only as an impurity.

9. Process for the preparation of a composition according to claim 1 to 8, **characterized in that** the composition is converted into a porous form, a porous solid or a porous granulate with the aid of a fluidized bed granulation.

10. Process for the preparation of a composition according to claim 9, **characterized in that** mixtures of gum arabic and pectins are used and such products have a fibrous structure.

11. Use of a composition according to claim 1 to 8, **characterized in that** the composition is used as a food additive, as a dietary supplement, as a dietary additive, as a dietary preparation, as a food additive for sportsmen, as a dietary supplement for sportsmen, as a baby food supplement, as an additive to baby food, for increase of physical performance or for preventive nutrition.

## Revendications

1. Composition qui contient des acides aminés et des polysaccharides et convient à une administration orale, **caractérisée en ce que** la matière sèche de la composition est constituée de
a) un composant d'acides aminés avec au moins un acide aminé ou avec un ou plusieurs acides aminés choisis dans le groupe L-glutamine, L-leucine, L-proline, L-lysine, L-valine, L-isoleucine, L-tyrosine, L-sérine, L-thréonine, L-alanine, L-phénylalanine, L-arginine, L-histidine, glycine, L-cystéine, L-cystine, acide L-glutamique, L-tryptophane, L-méthionine, L-asparagine et acide L-aspartique, lorsque le composant d'acides aminés est constitué d'acides aminés et que les acides aminés sont présents sous forme d'acides aminés libres ou sous forme de leurs sels,
b) un composant polysaccharidiqu constitué de gomme arabique et de pectine,
c) éventuellement une, plusieurs ou toutes les substances du groupe des substances L-carnitine, taurine ou choline, en tant que telles, sous forme de sel, de précurseur ou de dérivé, et
d) éventuellement un ou plusieurs auxiliaires ou additifs,
où le composant d'acides aminés est présente dans une fraction en poids d'au moins 50 pour cent en poids et le composant polysaccharidique est présente de 1 à 50 pour cent en poids, respectivement sur la base du matière sèche de la composition, et le
composant acide aminé et le composant polysaccharidique sont mélangés, le composant acide aminé et le composant polysaccharidique sont contenus sous forme de mélange ou dans un granulé.

2. Composition selon la revendication 1, **caractérisée en ce que** la composition est constituée d'au moins 95% en poids de le composant d'acides aminés et le composant polysaccharidique et éventuellement une ou plusieurs substances du groupe des substances L-carnitine, taurine ou choline, en tant que telles, sous forme de sel, de précurseur ou de dérivé, où le composant d'acides aminés est présente dans une fraction en poids de 50 à 90 pour cent en poids et les pourcentages en poids se rapportent à la matière sèche de la composition.

3. Composition selon l'une des revendications précédentes, **caractérisée en ce que** les auxiliaires et additifs sont présents dans la composition en une fraction pondérale de 0 à 5 pour cent en poids, les pourcentages en poids se rapportant à la matière sèche de la composition.

4. Composition selon l'une des revendications précédentes, **caractérisée en ce que** des arômes ou des substances colorantes sont contenues dans la composition à titre de auxiliaires et additifs.

5. Composition selon l'une des revendications précédentes, **caractérisée en ce que** le composant d'acides aminés est constituée des acides aminés L-glutamine, L-leucine, L-proline, L-lysine, L-valine, L-isoleucine, L-tyrosine, L-sérine, L-thréonine, L-alanine, L-phénylalanine, L-arginine, L-histidine, glycine, L-cystéine ou L-cystine ou L-cystéine et L-cystine, acide L-glutamique, L-tryptophane, L-méthionine, éventuellement de l'acide L-aspartique et éventuellement de la L-asparagine.

6. Composition selon l'une des revendications précédentes, **caractérisée en ce que** la composition contient 9 à 17,5% en poids de L-glutamine, 5 à 11% en poids de L-leucine, 3 à 7% en poids de L-proline, 2,6 à 6,6. % en poids de L-Lysine HCI, 1,9 à 6% en poids de L-valine, 1,7 à 5,7% en poids de L-isoleucine, 1,9 à 4,9% en poids de L-tyrosine, 1,7 à 4,7% en poids de L-sérine, 1,7 à 4,7% en poids L-thréonine, 1,1 à 4,1% en poids L-alanine, 1 à 4,1% en poids L-phénylalanine, 0,9 à 4% en poids L-arginine, 1,2 à 2% en poids L-histidine, 1,2 à 2,2% en poids de glycine, 1,2 à 2,3% en poids de L-cystéine, 0,7 à 1,9% en poids d'acide L-glutamique, 0,7 à 1,9% en poids de L-tryptophane, 0,2 à 1,4% en poids de L-méthionine et éventuellement 4 à 12 pour cent en poids de L-carnitine, 0,8 à 1,9 pour cent en poids de taurine et 1,5 à 2,5 pour cent en poids de choline, où les pourcentages de le poids est basé sur la matière sèche de la composition et la somme des pourcentages en poids donne une valeur allant jusqu'à 90 pour cent en poids.

7. Composition selon l'une des revendications précédentes, **caractérisée en ce que** la composition est solide, se présente sous forme de poudre, de granulés, de solution, de suspension, de dispersion liquide, gélifiée ou pâteuse ou contient un liquide ou de l'eau.

8. Composition selon l'une des revendications précédentes, **caractérisée en ce que** la composition contient des protéines, des peptides, des glucides digestibles, des édulcorants, des lipides, des vitamines, des substances minérales ou des minéraux pas du tout ou uniquement sous forme d'impureté.9. Procédé de préparation d'une composition selon les revendications 1 à 8, **caractérisé en ce que** la composition est transformée en une forme poreuse, un solide poreux ou un granulé poreux à l'aide d'une granulation en lit fluidisé.

9. Procédé de préparation d'une composition selon les revendications 1 à 8, **caractérisée en ce que** la composition est transformée en une forme poreuse, un solide poreux ou un granulé poreux à l'aide d'une granulation en lit fluidisé.

10. Procédé de préparation d'une composition selon la revendication 9, **caractérisée en ce que** des mélanges de gomme arabique et de pectines sont utilisés et de tels produits ont une structure fibreuse.

11. Utilisation d'une composition selon les revendications 1 à 8, **caractérisée en ce que** la composition est utilisée comme additif alimentaire, comme complément alimentaire, comme additif diététique, comme préparation diététique, comme additif alimentaire pour sportifs, comme diététique complément pour sportifs, comme complément alimentaire pour bébé, comme additif à l'alimentation pour bébé, pour augmenter les performances physiques ou pour la nutrition préventive.
